# EUROPÄISCHE PATENTANMELDUNG

(11) **EP 2 151 450 A1**
(43) Veröffentlichungstag der Anmeldung: **10.02.2010**
(21) Anmeldenummer: 08161348.1
(22) Anmeldetag: 29.07.2008
(51) Int. Cl.: C07K 1/14, C07K 7/64, C30B 7/06

(54) **Verfahren zur Aufarbeitung von mikrobiologisch hergestellten zyklischen Oligopeptiden**

(71) Anmelder: Sandoz AG, 4056 Basel (CH)
(72) Erfinder: Die Erfindernennung liegt noch nicht vor
(74) Vertreter: Englmeier, Ludwig

(57) **Zusammenfassung**

Die vorliegende Erfindung betrifft Verfahren zur Aufarbeitung von mikrobiologisch hergestellten, unpolaren, zyklischen Oligopeptiden umfassend den Schritt a) Extraktion des bei der mikrobiologischen Herstellung anfallenden Gesamt-Fermentationsbreis mit einem flüssigen, mit Wasser nicht mischbaren, etherhaltigen Extraktionsmittel, wobei die Menge des Extraktionsmittels ausreicht, um mit dem Gesamt-Fermentationsbrei ein Zwei-Phasen System zu bilden, sowie neue Solvate aus Cyclosporin A und Methyl-t-butylether.

## Beschreibung

Die vorliegende Erfindung betrifft Verfahren zur Aufarbeitung von mikrobiologisch hergestellten, unpolaren, zyklischen Oligopeptiden umfassend den Schritt
a) Extraktion des bei der mikrobiologischen Herstellung anfallenden Gesamt-Fermentationsbreis mit einem flüssigen, mit Wasser nicht mischbaren, etherhaltigen Extraktionsmittel, wobei die Menge des Extraktionsmittels ausreicht, um mit dem Gesamt-Fermentationsbrei ein Zwei-Phasen System zu bilden, sowie neue Solvate aus Cyclosporin A und Methyl-t-butylether.

Zyklische Oligopeptide, insbesondere Undekapeptide sind seit langem u. a. als mikrobiologische Stoffwechselprodukte bekannt. Von den Undekapeptiden haben insbesondere Cyclosporine Bedeutung erlangt.

Cyclosporine, insbesondere Cyclosporin A, sind nämlich wertvolle, pharmazeutische Wirkstoffe, die als Immunosuppressiva, insbesondere bei Organtransplantationen eingesetzt werden können. Darüber hinaus eignen sich Cyclosporine zur Behandlung von Erkrankungen, wie z. B. Diabetes und Psoriasis, sowie zahlreichen Autoimmunerkrankungen, wie z. B. rheumatischer Arthritis und chronischen Entzündungen. Cyclosporine, insbesondere Cyclosporin A, eignen sich auch aufgrund ihrer Hemmwirkung gegen das Humane-Immundefizienz-Virus (HIV) zur Bekämpfung von dadurch verursachten Erkrankungen. Außerdem konnte eine pharmakologische Wirkung von Cyclosporinen, insbesondere Cyclosporin A, zur Sensibilisierung von Krebszellen gegenüber Chemotherapeutika wie z. B Vincistin oder Daunorubicin festgestellt werden. Weiterhin können die neuroprotektiven bzw. regenerativen Eigenschaften von Cyclosporinen, insbesondere Cyclosporin A, bei verschiedenen neurologischen Indikationen, wie z. B. Alzheimer, Amyotropher Lateralsclerose oder Parkinson, eingesetzt werden.

Wie bereits erwähnt, können zyklische Undekapeptide, wie Cyclosporine, durch mikrobiologische Prozesse hergestellt werden.

Insbesondere kann Cyclosporin A mit Hilfe eines Stammes der Pilzspezies Cylindrocarpon lucidum Booth bzw. eines Stammes der Pilzspezies Tolypocladium inflatum Gams produziert werden. Dieser Stamm der Pilzspezies Tolypocladium wurde ursprünglich als ein Stamm der Pilzspezies Trichoderma polysporum bezeichnet, der beim United States Department of Agriculture (Northern Research and Development Division), Peoria, IL, USA unter der Nummer NRRL 8044, deponiert wurde. Der erstgenannte Stamm der Pilzspezies Cylindrocarpon lucidum Booth wurde ebenfalls beim United States Department of Agriculture (Northern Research and Development Division), Peoria, IL, USA unter der Nr. NRRL 5760 deponiert. Weitere Stämme zur mikrobiologischen Herstellung von Cyclosporinen, insbesondere Cyclosporin A, sind Stämme der Pilzspezies Tolypocladium geodes, Tolypocladium cylindrosporum und Tolypocladium sp. LeA3, wobei der letztgenannte Stamm gemäß dem Budapester Abkommen in der Depotstelle des Zentralbüros für Schimmelkulturen in Holland unter der Nummer CBS 630.92 deponiert ist.

Die großtechnische Herstellung von Cyclosporinen durch mikrobiologische Prozesse ist von großer Bedeutung. Aus diesem Grund hat es auch nicht an Versuchen gefehlt, die mikrobiologische Herstellung durch Auffindung und Züchtung effizienter Stämme der genannten Pilzspezia oder neuer Pilzspezia zu verbessern. Die vorstehend genannten Stämme sind Beispiele für diese Bemühungen. Bei einer großtechnischen, mikrobiologischen Herstellung von Cyclosporinen ist aber nicht nur ein effektiver Fermentationsprozess wichtig, sondern auch eine effektive Aufarbeitung der damit gewonnenen Stoffwechselprodukten.

Gemäß den aus z. B. DOS 2455859 bzw. DD-B-298276 bekannten Verfahren zur Isolierung von Cyclosporinen, insbesondere von Cyclosporin A, aus dem Gesamt-Fermentationsbrei, d. h. aus der Kulturbrühe, kann entweder die Biomasse mit Produkt von der Kulturflüssigkeit durch Filtrieren oder Zentrifugieren abgetrennt oder der Gesamt-Fermentationsbrei, d. h. ohne Abtrennung der Biomasse, aufgearbeitet werden.

Gemäß der ersten Verfahrensvariante wird die abgetrennte Biomasse mit den Stoffwechselprodukten einer vorzugsweise mehrmaligen Extraktion mit Methanol oder Aceton unterworfen und der abgetrennte Extrakt vorzugsweise bis zu einem wässrigen Rückstand eingeengt, um z. B. mit Ethylenchlorid oder Chloroform, vorzugsweise mehrmals extrahiert zu werden. Das von der Biomasse abgetrennte Kulturfiltrat wird vorzugsweise ebenfalls entsprechend extrahiert.

Gemäß der zweiten Verfahrensvariante, bei der die Biomasse nicht abgetrennt wird, wird die Kulturbrühe, d. h. der Gesamt-Fermentationsbrei, u. a. mit den vorstehend genannten Lösungsmitteln Ethylenchlorid oder Chloroform extrahiert und die abgetrennte, organische Phase nach Einengung mehrmals an unterschiedlichen, stationären Säulenfüllungen mit unterschiedlichen Elutionsmitteln zur weiteren Aufarbeitung chromatographiert. Dabei kommen Sephadex LH-20 als stationäres Säulenmaterial mit Methanol als Elutionsmittel bzw. Aluminiumoxid als Säulenmaterial mit Toluol/Ethylacetat als Elutionsmittel in Frage. Eine entsprechende chromatographische Aufarbeitung kann auch bei der erstgenannten Aufarbeitungsvariante erfolgen, wobei der aus den Ethylenchlorid-oder Chloroform- Extrakten gewonnene Rückstand mit Hilfe des Säulenmaterials Kieselgel in Kombination mit dem Elutionsmittel Chloroform und des Säulenmaterials Sephadex LH-20 in Kombination mit dem Elutionsmittel Methanol gereinigt und in die gewünschten Produkte, vorzugsweise Cyclosporin A, weiter aufgetrennt wird.

Gegenüber diesen vorbekannten Aufarbeitungsverfahren bestand die Aufgabe, technologisch vorteilhafte, umweltverträgliche und effiziente Aufarbeitungsverfahren für mikrobiologisch produzierte, zyklische Oligopeptide, insbesondere Undekapeptide wie Cyclosporine, mit einer sehr guten Ausbeutung und Reinheit der Produkte zur Verfügung zu stellen. Insbesondere bestand die Aufgabe daran, die Zahl der zum Einsatz kommenden organischen Lösungsmittel zu vermindern sowie deren Recyclierbarkeit zu erreichen, um so u. a. das Aufarbeitungsverfahren ökologisch effizienter zu gestalten.

Diese Aufgabe wird durch das Zurverfügungstellen des erfindungsgemäßen Verfahrens zur Aufarbeitung von mikrobiologisch hergestellten, unpolaren, zyklischen Oligopeptiden gelöst, umfassend den Schritt
a) Extraktion des bei der mikrobiologischen Herstellung anfallenden Gesamt-Fermentationsbreis mit einem flüssigen, mit Wasser nicht mischbaren, etherhaltigen Extraktionsmittel, wobei die Menge des Extraktionsmittels ausreicht, um mit dem Gesamt-Fermentationsbrei ein Zwei-Phasen System zu bilden.

Nach dem erfindungsgemäßen Verfahren können mikrobiologisch hergestellte, unpolare, zyklische Oligopeptide mit vorzugsweise 5 bis 15 Peptidbindungen aufgearbeitet werden. Der Begriff Oligopeptid ist erfindungsgemäß als organische Verbindung umfassend eine bestimmte Anzahl von Peptidbindungen zu verstehen, wobei lediglich vorzugsweise zwei aufeinander folgende Stickstoffatome des Ringsystems durch zwei dazwischen liegende Kohlenstoffatome getrennt sind. Bevorzugt können unpolare, zyklische Oligopeptide aus 8 bis 13 Aminosäuren, besonders bevorzugt Undekapeptide, mit Hilfe des erfindungsgemäßen Aufarbeitungsverfahrens isoliert werden. Dieses Verfahren eignet sich ganz besonders bevorzugt zur Aufarbeitung der mikrobiologisch hergestellten, unpolaren Undekapeptiden wie Cyclosporinen, ganz besonders bevorzugt Cyclosporin A.

Als unpolar sind erfindungsgemäß solche zyklische Oligopeptide, insbesondere Undekapeptide wie Cyclosporine zu verstehen, die als lipophile Verbindungen eine Wasserlöslichkeit von < 0,1 g/l Wasser bei 25 °C aufweisen.

Die mikrobiologische Herstellung der genannten Produkte mit Hilfe der vorstehend genannten Stämme bestimmter Pilzspezies sind dem Fachmann hinlänglich bekannt. Bereits in der deutschen Offenlegungsschrift 2455859 wird nicht nur die Kultivierung der Pilzstämme NRRL 5760 bzw. NRRL 8044 auf den Seiten 3-4 offenbart, sondern es geht auch der Fermentationsprozess zur Herstellung von Cyclosporinen aus den Seiten 4-5 bzw. aus Beispiel 1 hervor.

Dies gilt auch für die Kultivierung des Stamms Tolypocladium sp.LeA3, die aus den Seiten 3-5 von WO 94/16091 zu entnehmen ist, sowie für den Fermentationsprozess unter Einsatz dieses Pilzes zur Herstellung von Cyclosporinen, der den Beispielen 1 und 2 von WO 94/16091 zu entnehmen ist. Weitere mikrobiologische Verfahren sind in DD-B-298276, EP-A-0507968, US 5156960 u. a. offenbart.

Aus dem Kulturbrei, d. h. aus dem Gesamt-Fermentationsbrei, der bei der mikrobiologischen Herstellung anfällt, können erfindungsgemäß die gewünschten Produkte durch Extraktion gewonnen werden. Dazu wird der Gesamt-Fermentationsbrei mit einem flüssigen, mit Wasser nicht mischbaren, etherhaltigen Extraktionsmittel versetzt, wobei die Menge des Extraktionsmittels ausreicht, um mit dem Gesamt-Fermentationsbrei ein 2-Phasen System zu bilden.

Erfindungsgemäß wird unter einem flüssigen, mit Wasser nicht mischbaren Extraktionsmittel ein Extraktionsmittel verstanden, das bei 20 °C mit Wasser zumindest eine Mischungslücke aufweist, bei der das flüssige Extraktionsmittel mit dem Wasser zumindest so unvollständig mischbar bzw. unvollständig löslich ist, dass eine Phasentrennung auftritt.

Vorzugsweise weisen diese erfindungsgemäß zum Einsatz kommenden, etherhaltigen, mit Wasser nicht mischbare Extraktionsmittel eine Dichte von höchstens 0,9 g/cm³, vorzugsweise eine Dichte von 0,6 bis 0,85 g/cm³, besonders bevorzugt von 0,7 bis 0,8 g/cm³, jeweils gemessen bei 20 °C, auf. Vorzugsweise besteht das etherhaltige Extraktionsmittel im wesentlichen aus einem oder mehreren Ethern, ganz besonders bevorzugt aus wenigstens einem Ether der allgemeinen Formel

R₁-O-R₂

in der R₁ und R₂ unabhängig voneinander, für einen linearen oder verzweigten Alkylrest mit C₁ bis C₅ steht und mindestens einer der Reste R₁ und R₂ wenigstens 3 C-Atome, vorzugsweise wenigstens 4 C-Atome, aufweist.

Besonders bevorzugte Ether, die zur Extraktion verwendet werden können, ist wenigstens ein Ether ausgewählt aus der Gruppe umfassend Di-isopropylether, Di-n-propylether, Di-t-butylether, Di-iso-butylether, Methyl-t-butylether, Ethyl-t-butylether, Propyl-t-butylether und n-Butyl-t-butylether, wobei der Einsatz von Methyl-t-butylether besonders bevorzugt ist.

Ein weiterer Gegenstand der vorliegenden Erfindung ist daher auch die Verwendung von flüssigen, mit Wasser nicht mischbaren, etherhaltigen Extraktionsmittel, vorzugsweise mit einer Dichte von höchstens 0,9 g/cm³, gemessen bei 20 °C, als Mittel zur Extraktion von mikrobiologisch hergestellten, unpolaren, zyklischen Oligopeptiden, vorzugsweise Undekapeptiden, besonders bevorzugt Cyclosporinen, ganz besonders bevorzugt von Cyclosporin A aus dem bei der mikrobiologischen Herstellung anfallenden Gesamt-Fermentationsbrei. Bei dieser erfindungsgemäßen Verwendung kommen vorzugsweise die vorstehend aufgeführten Ether in solchen angegebenen Mengen zum Einsatz, dass sie mit dem Gesamt-Fermentationsbrei ein 2-Phasen-System bilden.

Vorzugsweise erfolgt die Extraktion des Gesamt-Fermentationsbreis, insbesondere bei der Isolierung und Aufarbeitung von Cyclosporinen, besonders bevorzugt von Cyclosporin A, bei einem pH-Wert von 7 bis 9, vorzugsweise 7,5 bis 8,5.

Zur Beschleunigung der Phasentrennung können bekannte wasserlösliche Netzmittel, z.B. auf Polyacrylatbasis, in einer ausreichenden Menge dem Gesamt-Fermentationsbrei zugesetzt werden.

Vorzugsweise wird die Extraktion mehrmals durchgeführt. Besonders bevorzugt wird der Gesamt-Extraktionsbrei zweimal mit dem etherhaltigen Extraktionsmittel extrahiert.

Die Extraktion des Gesamt-Fermentationsbreis erfolgt vorzugsweise mit dem etherhaltigen, mit Wasser nicht mischbaren Extraktionsmittel bei Raumtemperatur, besonders bevorzugt bei 20 bis 30 °C.

Die Extraktion des Gesamt-Fermentationsbreis kann in üblichen Apparaturen durchgeführt werden, wobei die bei der Extraktion gewonnenen Extrakte nach Abtrennung der wässrigen Phase mit der Biomasse zur weiteren Aufarbeitung vereinigt werden.

Vorzugsweise schließt sich daher an den Verfahrensschritt a) ein weiterer Verfahrensschritt b) an, gemäß dem der im Extrakt vorliegende Restwassergehalt auf weniger als 1 Gew.%, vorzugsweise auf weniger als 0,3 Gew.% erniedrigt wird. Bevor diese Erniedrigung des Restwassergehaltes durchgeführt wird, wird vorzugsweise der Extrakt mit einer wässrigen Lösung, vorzugsweise mit Wasser, gewaschen, um ggfs. vorhandene Reste an Biomasse zu entfernen. Zur Erniedrigung des Restwassersgehaltes eines so ggfs. gereinigten Extraktes kann eine azeotrope Destillation durchgeführt werden. Dabei wird nicht nur der Restwassergehalt auf das gewünschte Maß in dem Extrakt erniedrigt, sondern auch die Konzentration der mikrobiologisch hergestellten Produkte, vorzugsweise der Cyclosporine, besonders bevorzugt des Cyclosporin A, erhöht. Bei dieser Konzentrierung wird vorzugsweise ein Cyclosporingehalt von 10 bis 35 Gew.% eingestellt.

Durch die azeotrope Destillation in Schritt b) gelingt es, nicht nur den Restwassergehalt im Extrakt auf den gewünschten, niedrigen Wassergehalt einzustellen, sondern auch eine für die anschließende Kristallisation des aufgearbeiteten Produktes vorteilhafte Konzentration des Extraktes einzustellen, wobei das Produkt vorzugsweise als Cyclosporin-Ether-Solvat kristallisiert wird. Es wurde nämlich gefunden, dass die Kristallisation von Cyclosporinen als Cyclosporin-Ether-Solvat, und insbesondere die Kristallisation von Cyclosporin A als Cyclosporin A-Ether-Solvat, bei einem Restwassergehalt von weniger als 1 Gew.%, insbesondere von weniger als 0,3 Gew.%, deutlich besser vonstatten geht und leicht filtrierbare, praktisch farblose, weiße Cyclosporin-Ether-Solvat-Kristalle zugänglich macht. Die leichte Filtrierbarkeit der bereits sehr reinen Cyclosporin-Ether-Solvat-Kristalle erleichtert und verkürzt das Gesamtaufarbeitungsverfahren zusätzlich.

Dementsprechend umfasst das erfindungsgemäße Verfahren zur Aufarbeitung von mikrobiologisch hergestellten Produkten, vorzugsweise Cyclosporinen, besonders bevorzugt von Cyclosporin A, einen weiteren Verfahrensschritt c), gemäß dem der im Schritt b) aufkonzentrierte, von Restwassergehalt weitgehend befreite Extrakt durch Abkühlen auf Temperaturen von -10 °C bis 15 °C, vorzugsweise auf Temperaturen von -5 °C bis 10 °C, zur Kristallisation als Ethersolvatprodukt, vorzugsweise Cyclosporin-Ether-Solvat, abgekühlt wird. Vorzugsweise ist für das Auskristallisieren der Cyclosporin-Ether-Solvat-Kristalle keine Zugabe eines organischen Lösungsmittels, in dem die Kristalle nicht löslich sind, zu dem konzentrierten, vom Restwassergehalt weitgehend befreiten Extrakt notwendig, da gut filtrierbare, praktisch farblose, weiße Kristalle bereits durch das Abkühlen des konzentrierten Extraktes erhalten werden. Gegebenenfalls können die Kristalle, vorzugsweise die Cyclosporin-Ether-Solvat-Kristalle mit einem organischen Lösungsmittel, in dem sich die Kristalle nur langsam lösen, zur weiteren Reinigung gewaschen werden. Gegebenenfalls können die Cyclosporin-Ether-Solvat-Kristalle durch bekannte chromatographische Aufreinigung und Kristallisation in geeigneten, bekannten Lösungsmitteln in die Cyclosporine A-Z aufgetrennt werden (siehe dazu auch WO97/46575, EP0725076, EP0888382).

Dementsprechend ist ein weiterer Gegenstand der vorliegenden Erfindung die Verwendung von kristallinen Cyclosporin-Ether-Solvaten bei der großtechnischen Herstellung von Cyclosporinen, vorzugsweise von Cyclosporin A. Für diese großtechnische Herstellung von Cyclosporinen eignen sich insbesondere die erfindungsgemäß erhaltenen, kristallinen Cyclosporin-Ether-Solvat-Kristalle. Überaschenderweise ermöglicht die Extraktion des Gesamtfermentationsbreis mit dem erfindungsgemäßen etherhaltigen Exatraktionsmittel, insbesondere in Verbindung mit dem Schritt der Erniedrigung des Restwassergehaltes, die Kristallisation von Cyclosporin-Ether-Solvat-Kristallen, die gute Filtrierbarkeit und hohe Reinheit aufweisen. Dadurch wird das Gesamtaufarbeitungsverfahren deutlich vereinfacht.

Eine besonders bevorzugte Ausführungsform der vorliegenden Erfindung ist ein Verfahren zur Aufarbeitung von mikrobiologisch hergestellten, unpolaren Cyclosporinen, vorzugsweise von Cyclosporin A, umfassend die Schritte
a) Extraktion des bei der mikrobiologischen Herstellung von Cyclosporinen anfallenden Gesamt-Fermentationsbreis mit einem flüssigen, mit Wasser nicht mischbaren etherhaltigen Extraktionsmittel, wobei die Menge des Extraktionsmittels ausreicht, um mit dem Gesamt-Fermentationsbrei ein 2-Phasen System zu bilden,
b) Erniedrigen des im Extrakt gelösten Restwassergehaltes auf weniger als 1 Gew.%, vorzugsweise auf weniger als 0,3 Gew.%, wobei der im Schritt a) erhaltene Extrakt ggfs. vor dieser Erniedrigung des Restwassergehalts mit einer wässrigen Lösung gewaschen werden kann, und
c) Kristallisieren der Cyclosporine als Cyclosporin-Ether-Solvate, wobei vorzugsweise vor der Kristallisation der im Schritt b) erhaltene Extrakt auf einen Cyclosporingehalt von 10 bis 25 Gew.%, bezogen auf den Gesamt-Extrakt, aufkonzentriert wird und zur Kristallisation der aufkonzentrierte Extrakt auf Temperaturen von -10 °C bis 15 °C, vorzugsweise -5 °C bis 10 °C, abgekühlt wird.

Die vorstehend angegebenen Reaktionsbedingungen für die einzelnen Verfahrensschritte gelten ebenso für die erfindungsgemäß bevorzugte Aufarbeitung von unpolaren Cyclosporinen, die mikrobiologisch hergestellt worden sind, insbesondere zur der Aufarbeitung und Isolierung von Cyclosporin A. Dies gilt auch für die zum Einsatz kommenden etherhaltigen Extraktionsmittel, vorzugsweise Ether der vorstehend angegebenen allgemeinen Formel. Dementsprechend können mit dem bevorzugten erfindungsgemäßen Aufarbeitungsverfahren leicht filtrierbare, praktisch farblose, weiße Cyclosporin-Ether-Solvat-Kristalle erhalten werden.

Diese Kristalle eignen sich insbesondere auch, um durch weiteres Umkristallisieren in bekannter Weise und/oder durch chromatographische Aufarbeitung mit bekannten Säulenmaterialien in Kombination mit bekannten, geeigneten Eluierungsmittel in die Cyclosporine A-Z aufgetrennt zu werden.

Es wurde beobachtet, dass in Cyclosporin A-Ether-Solvat-Kristallen das ansonsten schwer abtrennbare Isocyclosporin A deutlich abgereichert ist.

Dementsprechend ist ein weiterer Gegenstand der vorliegenden Erfindung Cyclosporin A mit einem Gehalt von weniger als 0,05 Gew.% Isocyclosporin A, vorzugsweise mit einem Gehalt von 0,04 bis 0,05 Gew.% Isocyclosporin A.

Das Cyclosporin A, das nach dem erfindungsgemäßen Aufarbeitungsverfahren erhältlich ist, zeichnet sich auch durch einen geringeren Gehalt an Cyclosporin H und Cyclosporin T aus.

Ein weiterer Gegenstand der vorliegenden Erfindung ist daher auch Cyclosporin A, wobei dessen Gesamtgehalt an Cyclosporin H und Cyclosporin T weniger als 0,5 Gew.%, vorzugsweise von 0,1 bis 0,4 Gew.% beträgt.

Ebenso ist ein weiter Gegenstand der vorliegenden Erfindung Cyclosporin A mit einem Gehalt von 0,04 bis 0,005 Gew.% Isocyclosporin A, wobei der zusätzliche Gesamtgehalt an Cyclosporin H und Cyclosporin T von 0,01 bis 4 Gew.% beträgt. Vorzugsweise ist dieses Cyclosporin A mit den geringen Gehalten an Nebenprodukten durch die erfindungsgemäße Aufarbeitung und Weiterbehandlung von erfindungsgemäß erhaltenen Cyclosporin A-Methyl-t-Butyl-Ether-Solvat-Kristallen erhältlich.

Darüberhinaus gelingt es, mit Hilfe des erfindungsgemäßen, bevorzugten Aufarbeitungsverfahren von unpolaren, mikrobiologisch hergestellten Cyclosporinen, die Cyclosporin-Ether-Solvat-Kristalle nicht nur mit einer ausgezeichneten Reinheit und mit Ausbeuten über 90 %, vorzugsweise ≥ 95 %, zu erhalten, sondern auch mit einer sehr guten Handhabbarkeit der Kristalle, da wenigstens 90 % der Kristalle eine Kristallgröße > 10 µm aufweisen und der Median der Korngrößenverteilung bei etwa 60 µm liegt. Zur Messung der Korngrößenverteilung wurden 3 x 1 g der Probe in 60 mL Wasser dispergiert und nach Zugabe der Dispergiereinheit Hydro 2000S bei einer Rührgeschwindigkeit von 2000 rpm mit dem Messgerät "Mastersizer 2000" der Fa. Malvern gemessen. Zur Auswertung wurde das Modell "Fraunhofer" des Geräteherstellers verwendet. Die oben angeführten Werte verstehen sich als Mittelwerte über 3 solcher Messungen.

Ein weiterer Gegenstand der vorliegenden Erfindung ist daher kristallines Cyclosporin A-Methyl-t-Butylether-Solvat, von dem wenigstens 90 % der Kristalle eine Kristallgröße > 10 µm aufweisen, wobei vorzugsweise der Median der Größenverteilung zwischen 30µm und 100µm liegt, vorzugsweise zwischen 40µm und 80µm.

Wie bereits ausgeführt eignet sich das erfindungsgemäße Verfahren insbesondere zur Aufarbeitung von mikrobiologisch hergestelltem Cyclosporin A als kristallines Cyclosporin A-Methy-t-Butylether-Solvat. Solche Kristalle konnten mit Hilfe von Röntgenpulverdiffraktogrammen eindeutig bestimmt werden.

Röntgenpulverdiffraktogramme (XRPD) wurden auf einem AXS-Bruker Röntgenpulverdiffraktometer D-8 unter Verwendung der folgenden Aufnahmeparameter bei Umgebungsbedingungen gewonnen: Röhrenanode: Cu; Generatorspannung: 40kV; Generatorstrom: 40 mA; Anfangswinkel: 2° 2-Theta; Endwinkel: 40° 2-Theta; Schrittgröße: 0.01 ° 2-Theta; Zeit pro Schritt: 2 Sekunden. Die typische Genauigkeit der 2-theta Werte liegt im Bereich von ± 0.1 ° 2-Theta. Daher kann ein Diffraktionspeak (Scheitelpunkt) der bei 5.0° 2-Theta verzeichnet ist, zwischen 4.9 and 5.1° 2-Theta auf den meisten Röntgendiffraktometern bei Standardbedingungen erscheinen.

Ein weiterer Gegenstand der vorliegenden Erfindung ist daher kristallines Cyclosporin A-Methyl-t-Butylether-Solvat, das ein Röntgenpulverdiffraktogramm umfassend Scheitelpunkte (peaks) bei 2 Theta Winkeln von 7,0° +/- 0,1 °, 8,2° +/-0,1°, 11,0° +/- 0,1° und 20,5° +/- 0,1 ° aufweist, insbesondere zusätzlich umfassend Scheitelpunkte bei 2 Theta Winkeln von 7,3°+/-0,1°, 11,8° +/- 0,1°, 13,3° +/- 0,1° und 16,5° +/- 0,1°.

Die Erfindung bezieht sich auch auf folgende Gegenstände:
1. Ein Verfahren zur Aufarbeitung von mikrobiologisch hergestellten, unpolaren, zyklischen Oligopeptiden umfassend den Schritt
   a) Extraktion des bei der mikrobiologischen Herstellung anfallenden Gesamt-Fermentationsbreis mit einem flüssigen, mit Wasser bei 25 °C nicht mischbaren, etherhaltigen Extraktionsmittel, wobei die Menge des Extraktionsmittels ausreicht, um mit dem Gesamt-Fermentationsbrei ein Zwei-Phasen System zu bilden.
2. Ein Verfahren nach Gegenstand 1, wobei die unpolaren, zyklischen Oligopeptide unpolare Undekapeptide sind.
3. Ein Verfahren nach Gegenstand 2, wobei die unpolaren Undekapeptide Cyclosporine, vorzugsweise Cyclosporin A, sind.
4. Ein Verfahren nach einem der Gegenstände 1 bis 3, wobei das etherhaltige Extraktionsmittel im wesentlichen aus einem oder mehreren Ether(n) besteht.
5. Ein Verfahren nach Gegenstand 3 oder 4, wobei das etherhaltige Extraktionsmittel im wesentlichen aus einem oder mehreren Ether(n) besteht und das Verfahren den weiteren Schritt umfasst:
   b) Erniedrigen des im Extrakt gelösten Restwassergehaltes auf weniger als 1Gew.%, vorzugsweise auf weniger als 0,3 Gew.%.
6. Ein Verfahren nach Gegenstand 5, wobei Schritt b) durch azeotrope Destillation erfolgt.
7. Ein Verfahren nach Gegenstand 5 oder 6, wobei der in Schritt a) erhaltene Extrakt vor der Weiterverarbeitung in Schritt b) mit einer wässrigen Lösung gewaschen wird.
8. Ein Verfahren nach einem der Gegenstände 5 bis 7, wobei der cyclosporinhaltige Extrakt auf einen Cyclosporingehalt von 10 Gew.% - 35 Gew.%, bezogen auf die Gesamt-Extraktmasse, aufkonzentriert wird.
9. Ein Verfahren nach einem der Gegenstände 1 bis 8, wobei als Ether wenigstens ein Ether der allgemeinen Formel

   R₁-O-R₂

   eingesetzt wird,
   in dem R₁ und R₂, unabhängig voneinander, für einen linearen oder verzweigten Alkylrest mit C₁ bis C₅ steht und mindestens einer der Reste R₁ oder R₂ wenigstens 3 C-Atome, vorzugsweise wenigstens 4 C-Atome aufweist.
10. Ein Verfahren nach Gegenstand 9 wobei als Ether wenigstens ein Ether ausgewählt aus der Gruppe umfassend Di-isopropylether, Di-n-propylether, Di-t-butylether, Di-iso-butylether, Methyl-t-butylether, Ethyl-t-butylether, Propyl-t-butylether und n-Butyl-t-butylether verwendet wird.
11. Ein Verfahren nach einem der Gegenstände 5 bis 10, wobei das Verfahren den weiteren Schritt umfasst:
   c) Kristallisieren des Cyclosporins als Cyclosporin-Ether-Solvat.
12. Ein Verfahren nach Gegenstand 11, wobei man für Schritt c) den aufkonzentrierten Extrakt auf eine Temperatur von -10°C bis 15°C abkühlt.
13. Ein Verfahren nach Gegenstand 11 oder 12, wobei die nach Schritt c) erhaltenen Cyclosporin-Ether-Solvat-Kristalle mit organischen Lösungsmitteln, in denen sich die Cyclosporin-Ether-Solvat-Kristalle nur langsam lösen, gewaschen werden.
14. Ein Verfahren nach einem der Gegenstände 1 bis 13, wobei der Gesamt-Fermentationsbrei mehrmals, vorzugsweise zweimal, mit dem etherhaltigen Extraktionsmittel extrahiert wird.
15. Ein Verfahren nach einem der Gegenstände 1 bis 14, wobei der Gesamt-Fermentationsbrei bei der Extraktion einen pH-Wert von 7 bis 9, vorzugsweise 7,5 bis 8,5 aufweist.
16. Ein Verfahren nach einem der Gegenstände 1 bis 15, **dadurch gekennzeichnet, dass** die Extraktion mit dem etherhaltigen Extraktionsmittel bei Raumtemperatur, vorzugsweise bei 20 bis 30 °C, durchgeführt wird.
17. Ein Verfahren nach einem der Gegenstände 11 bis 16, **dadurch gekennzeichnet, dass** das Cyclosporin-Ether-Solvat durch Umkristallisieren und/oder chromatographische Aufarbeitung jeweils in Cyclosporin A - Z aufgetrennt wird.
18. Kristallines Cyclosporin A - Methyl-t-butylether - Solvat, das ein Röntgenpulverdiffraktogramm umfassend Scheitelpunkte (peaks) bei 2 Theta Winkeln von 7,0° +/- 0,1 °, 8,2° +/- 0,1 °, 11,0° +/- 0,1 ° und 20,5° +/- 0,1 ° aufweist, insbesondere zusätzlich umfassend Scheitelpunkte bei 2 Theta Winkeln von 7,3° +/- 0,1°, 11,8° +/- 0,1°, 13,3° +/- 0,1° und 16,5° +/- 0,1°.
19. Kristallines Cyclosporin A - Methyl-t-butylether - Solvat, worin wenigstens 90% der Kristalle eine Kristallgröße größer als 10 µm haben (gemessen mittels Laserbeugung, Malvern Mastersizer 2000).
20. Cyclosporin A mit einem Gehalt von weniger als 0,05 Gew.% Isocyclosporin A, vorzugsweise mit einem Gehalt von 0,04 - 0,005 Gew.% Isocyclosporin A.
21. Cyclosporin A, wobei dessen Gesamtgehalt an Cyclosporin H und Cyclosporin T weniger als 0,5 Gew.%, vorzugsweise von 0,1 - 0,4 Gew.% beträgt.
22. Cyclosporin A mit einem Gehalt von 0,04 - 0,005 Gew.% Isocyclosporin A, wobei der zusätzliche Gesamtgehalt an Cyclosporin H und Cyclosporin T von 0,1 - 0,4 Gew.% beträgt.
23. Verwendung von kristallinem Cyclosporin-Ether-Solvat bei der großtechnischen Herstellung von Cyclosporinen.
24. Verwendung nach Gegenstand 23, um die Nebenprodukte Isocyclosporin A, Cyclosporin H und Cyclosporin T abzureichern.
25. Verwendung eines flüssigen, mit Wasser bei 25 °C nicht mischbaren, etherhaltigen Extraktionsmittels zur Extraktion von bei der mikrobiologischen Herstellung anfallenden, unpolaren, zyklischen Oligopeptiden, vorzugsweise Cyclosporinen, besonders bevorzugt von Cyclosporin A, aus dem Gesamt-Fermentationsbrei.

### Beschreibung der Figuren:

Figur 1: Röntgenpulverdiffraktogramm von kristallinem Cyclosporin A-Methyl-t-Butylether-Solvat
Figur 2: Infrarotspektrum von kristallinem Cyclosporin A-Methyl-t-Butylether-Solvat

### Beispiele

Die folgenden Beispiele beschreiben besondere Ausführungsformen der Erfindung detailliert, sie sind aber nicht als Beschränkung der Erfindung auszulegen.

### Beispiel 1: Extraktion von Cyclosporin A aus Fermentationsbrei

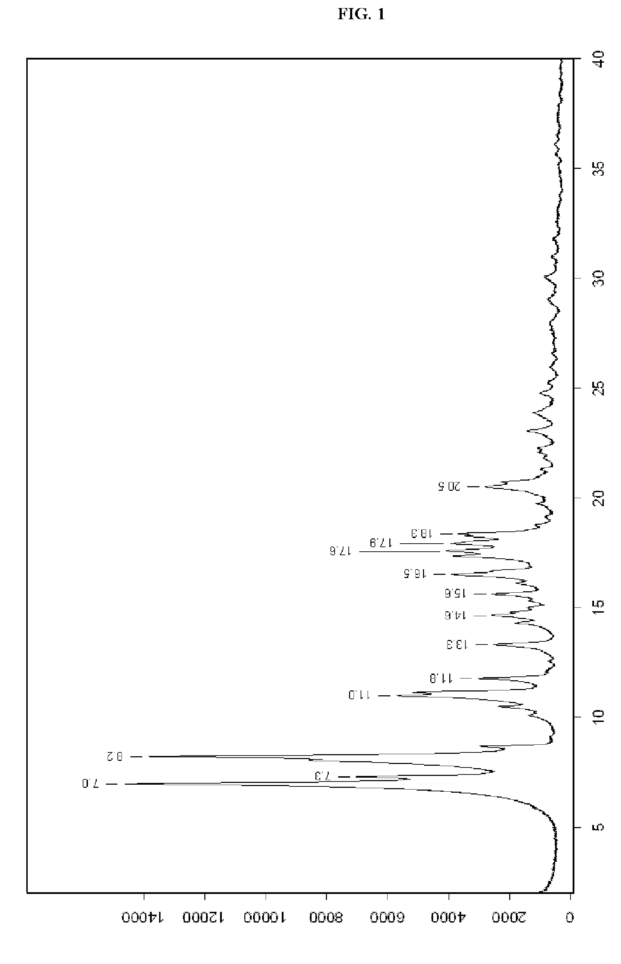

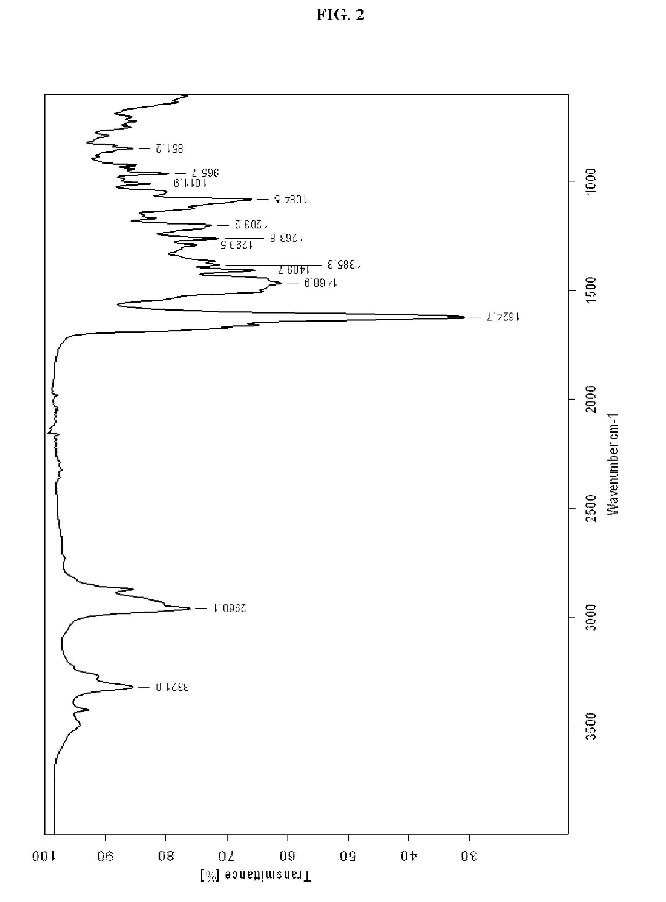

2000g Cyclosporin A-haltiger Fermentationsbrei, wie er z.B. auch durch Fermentation von *Tolypocladium inflatum* erhalten werden kann, wurden zweimal mit je 2000ml Methyl-tert.-butylether (MTBE) in Gegenwart von 4000 ppm des Polyacrylats Clariant MD07/049 extrahiert. Die Etherphasen wurden im Scheidetrichter abgetrennt und vereinigt. Die Cyclosporin A-haltige Etherphase wurde einmal mit 200 ml Wasser, das einen pH von etwa 7 aufwies, im Scheidetrichter gewaschen und die Etherphase wurde nach Filtration durch azeotrope Destillation bei einer Temperatur von etwa 53°C eingeengt, sodass eine Cyclosporinkonzentration von 150g/kg erreicht wurde. Die Temperatur wurde dann auf einen Wert knapp unter dem Siedepunkt abgesenkt und 2 Stunden lange bei dieser Temperatur gehalten, wobei über den Destillationskopf entweichendes MTBE durch frisches ersetzt wurde. Danach wurde die Lösung unter Normaldruck bei 53°C aufkonzentriert, sodass eine Cyclosporinkonzentration von 300 g/kg erreicht wurde. Der Restwassergehalt, der vor der azeotropen Destillation bei etwa 1,4 Gew.% lag, lag nun unter 0,1 Gew.%. Die Lösung wurde vorsichtig abgekühlt, und zwar in 120 Minuten von 53°C auf 45°C, in weiteren 60 Minuten auf 40°C, in weiteren 60 Minuten auf 30°C und in weiteren 60 Minuten auf 0°C. Man erhielt weißes, kristallines Cyclosporin A - MTBE Solvat in einer theoretischen Gesamtausbeute von etwa 82%. Ein Infrarotspektrum der erhaltenen Kristalle ist in Figur 2, ein Pulverröntgendiffraktogramm (XRPD) der erhaltenen Kristalle ist in Figur 1 gezeigt. Die Auswertung am Interferenzmikroskop ergab hochkristallines Material mit großen, doppelbrechenden Kristallen, mit einer mittleren Korngröße von etwa 65 µm.

### Beispiel 2: Großtechnische Extraktion von Cyclosporin A aus Fermentationsbrei

510kg Cyclosporin A-haltiger Fermentationsbrei wurden zweimal mit je 510I Methyl-tert.-butylether (MTBE) extrahiert, wobei 2,6kg des Polyacrylats Clariant MD07/049 zugegeben wurden. Die Etherphasen wurden in einem 300I Separator im Durchlauf abgetrennt und vereinigt. Die Cyclosporin A-haltige Etherphase wurde einmal mit 700I Reinwasser, das einen pH von etwa 7 aufwies, gewaschen, die Phasen im Separator wieder getrennt und die Etherphase wurde durch azeotrope Destillation in einem 1000I Dünnschichtverdampfer bei einer Temperatur von etwa 53°C eingeengt, sodass eine Cyclosporinkonzentration von 100 g/kg erreicht wurde. Die Temperatur wurde dann auf einen Wert knapp unter dem Siedepunkt abgesenkt und 2 Stunden lange bei dieser Temperatur gehalten, wobei über den Destillationskopf entweichendes MTBE durch frisches ersetzt wurde. Danach wurde die Lösung unter Normaldruck bei 53°C aufkonzentriert, sodass eine Cyclosporinkonzentration von 300 g/kg erreicht wurde. Der Restwassergehalt, der vor der azeotropen Destillation bei etwa 1,4 Gew.% lag, lag nun unter 0,1 Gew.%. Die Lösung wurde nun vorsichtig abgekühlt, und zwar in 120 Minuten von 53°C auf 45°C, in weiteren 60 Minuten auf 40°C, in weiteren 60 Minuten auf 30°C und in weiteren 60 Minuten auf 0°C. Man erhielt 8,55 kg weißes, kristallines Cyclosporin A - MTBE Solvat in einer theoretischen Gesamtausbeute von etwa 87%. Der Gehalt an Isocyclosporin A betrug 0,008 Gew.%, der Gesamtgehalt an Cyclosporin H und Cyclosporin T betrug 0,28 Gew.%, was gegenüber einem Extraktionsverfahren mit Butylacetat eine hochsignifikante Verringerung des Gehalts an diesen Verunreinigungen bedeutete.

## Patentansprüche

1. Ein Verfahren zur Aufarbeitung von mikrobiologisch hergestellten, unpolaren, zyklischen Oligopeptiden umfassend den Schritt
a) Extraktion des bei der mikrobiologischen Herstellung anfallenden Gesamt-Fermentationsbreis mit einem flüssigen, mit Wasser bei 25 °C nicht mischbaren, etherhaltigen Extraktionsmittel, wobei die Menge des Extraktionsmittels ausreicht, um mit dem Gesamt-Fermentationsbrei ein Zwei-Phasen System zu bilden.

2. Ein Verfahren nach Anspruch 1, wobei das unpolare, zyklische Oligopeptid Cyclosporin A ist.

3. Ein Verfahren nach Anspruch 1 oder 2, wobei das etherhaltige Extraktionsmittel im wesentlichen aus Methyl-tert.-Butylether besteht und das Verfahren den weiteren Schritt umfasst:
b) Erniedrigen des im Extrakt gelösten Restwassergehaltes auf weniger als 1 Gew.%, vorzugsweise auf weniger als 0,3 Gew.%, stärker bevorzugt auf weniger als 0,15 Gew.%.

4. Ein Verfahren nach Anspruch 3, wobei Schritt b) durch azeotrope Destillation erfolgt.

5. Ein Verfahren nach einem der Ansprüche 3 bis 4, wobei das unpolare, zyklische Oligopeptid Cyclosporin A ist und das Verfahren den weiteren Schritt umfasst:
c) Kristallisieren des Cyclosporins als Cyclosporin A- Methyl-tert.-Butylether-Solvat.

6. Kristallines Cyclosporin A - Methyl-t-butylether - Solvat, das ein Röntgenpulverdiffraktogramm umfassend Scheitelpunkte (peaks) bei 2 Theta Winkeln von 7,0° +/- 0,1°, 8,2° +/- 0,1°, 11,0° +/- 0,1° und 20,5° +/- 0,1° aufweist, insbesondere zusätzlich umfassend Scheitelpunkte bei 2 Theta Winkeln von 7,3° +/- 0,1°, 11,8° +/- 0,1°, 13,3° +/- 0,1° und 16,5° +/- 0,1°.

7. Kristallines Cyclosporin A - Methyl-t-butylether - Solvat, worin wenigstens 90% der Kristalle eine Kristallgröße größer als 10 µm haben.

8. Cyclosporin A mit einem Gehalt von 0,04 - 0,005 Gew.% Isocyclosporin A, wobei der zusätzliche Gesamtgehalt an Cyclosporin H und Cyclosporin T von 0,1 - 0,4 Gew.% beträgt.

9. Verwendung von kristallinem Cyclosporin-Ether-Solvat bei der großtechnischen Herstellung von Cyclosporinen.

10. Verwendung nach Anspruch 9, um die Nebenprodukte Isocyclosporin A, Cyclosporin H und Cyclosporin T abzureichern.
